# EUROPÄISCHE PATENTSCHRIFT

(11) **EP 4 193 926 B1**
(45) Veröffentlichungstag und Bekanntmachung des Hinweises auf die Patenterteilung: **05.08.2026**
(21) Anmeldenummer: 21856325.2
(22) Anmeldetag: 28.06.2021
(51) Int. Cl.: A61B 5/349, A61H 39/02, G16H 50/70, A61B 5/01, A61B 5/024, A61B 5/318, A61B 5/00, A61B 5/352

(54) **QUANTITATIVE BEURTEILUNG DER AKTIVITÄT VON AKUPUNKTURKANÄLEN**
QUANTITATIVE ASSESSMENT OF ACTIVITY OF ACUPUNCTURE CHANNELS
ESTIMATION QUANTITATIVE DE L'ACTIVITÉ DE CANAUX D'ACUPUNCTURE

(30) Priorität: 11.08.2020 RU 2020126806
(43) Veröffentlichungstag der Anmeldung: 14.06.2023
(73) Patentinhaber: Muzhikov, Valery Gennadievich, St. Petersburg 195220 (RU); Muzhikov, Ruslan Valerevich, St. Petersburg 195220 (RU)
(72) Erfinder: MUZHIKOV, Valery Gennadievich, St. Petersburg 195220 (RU)
(74) Vertreter: Jeck, Jonathan
(86) Internationale Anmeldenummer: PCT/RU2021/000268
(87) Internationale Veröffentlichungsnummer: WO 2022/035349

(56) Entgegenhaltungen:
- DE-A1- 19 603 190
- DE-B4- 19 983 992
- RU-C1- 2 569 246
- RU-U1- 86 870
- US-A1- 2007 049 888

## Beschreibung

### Bereich der Technologie

Die vorgeschlagene Erfindung bezieht sich auf die Medizin. Sie bezieht sich insbesondere auf die Akupunkturdiagnose und ermöglicht die Überwachung des Zustands der Akupunkturkanäle auch im Dauerüberwachungsmodus durch Analyse der Phasen- und Spektralkomponenten der Pulswelle.

In der modernen Fußreflexzonenmassage wird der Akabane-Thermodiagnosetest häufig zur Überwachung des Körperzustands eingesetzt. Der Test ist von der modernen Wissenschaft anerkannt und basiert auf der thermischen Beeinflussung der symmetrischen AT-Endpunkte (Akupunkturpunkte) der Ein- und Ausgänge der auptkanäle (Meridiane), um die Schwellenwerte der Temperaturschmerzempfindlichkeit des AT zu messen und die erhaltenen Daten weiter auszuwerten (Portnov F. G. Electro-puncture reflexotherapy. - Riga: Zinante, 1998.- P.103. Muzhikov V.G. Theorie und Praxis der Thermopunktur Kanal Diagnostik und Behandlung. - SPb.: OOO "Petrovsky Fund", 2000,- S.27-37).

Der Akabane-Test erfordert jedoch besondere Kenntnisse. Der Test ist zeitaufwändig und mit Schmerzen verbunden, um die Schwellenwerte der Temperaturempfindlichkeit zu ermitteln.

Es ist bekannt, dass chinesische Heiler seit dem Altertum die Aktivität der ACs (Akupunkturkanäle) bestimmen. Über diese Kanäle wird der Zustand einer Person auch durch die "Pulsdiagnose" ermittelt. Zu diesem Zweck wurden an beiden Händen des Patienten 3 Heilerfinger auf jedes Handgelenk im Bereich der Arteria radialis gelegt. Auf diese Weise wurden der Rhythmus und die Spannung der Pulswelle bewertet. Die Aktivität der 12 wichtigsten Kanäle wurde auf dieser Grundlage bewertet. Durch sie wurde eine allgemeine Diagnose des Zustands des Körpers erstellt. Diese Methode ist jedoch subjektiv und erfordert eine jahrelange Ausbildung und Praxis. Das ist nicht für jeden zugänglich.

Gegenstand der vorgeschlagenen Erfindung ist ein Verfahren zur quantitativen Bewertung der AK-Aktivität durch Auswertung von Phasen- und Spektralindizes der Pulswelle und des EKGs.

Ein weiterer Gegenstand der vorgeschlagenen Erfindung ist eine Vorrichtung, die die Durchführung einer solchen Bewertung ermöglicht.

### Stand der Technik.

Bekannt ist die "Methode der computergestützten Expressdiagnostik des Zustandes des menschlichen Organismus nach dem Kanon der tibetischen Medizin" (Patent RF 2264161, Datum des Beginns der Patentgültigkeit: 08.09.2003, veröffentlicht: 20.11.2005, ¹ 32).

Bei einem bekannten Patent wird die Anamnese der inneren Organe des Patienten in einen Computer eingegeben. Dabei wird der Kanon der tibetischen Medizin über den Zustand der drei Anfänge berücksichtigt. Die Eingeweide werden als "Wind", "Galle" und "Schleim" interpretiert. Bei der Bearbeitung jeder Gruppe von Merkmalen wird die höchste Punktzahl ausgewählt. Die Codierung erfolgt nach einem Drei-Punkte-System. Der Puls wird an sechs Punkten der Arteria radialis untersucht: links "tsong", "kan", "chag" und rechts "tsong", "kan", "chag". Dann wird der Wert des Impulssignalkoeffizienten in konventionellen Einheiten berechnet und die Anamnesedaten mit dem erhaltenen Wert des Impulssignalkoeffizienten an den untersuchten Punkten verglichen. Die gewonnenen Daten werden zur Beurteilung des Zustands des Patienten herangezogen. Die Methode verkürzt die Untersuchungszeit.

Um die bekannte Methode jedoch anwenden zu können, muss der Bediener die anamnestischen Daten für jede Untersuchung in Punkten bewerten. Damit ist es nicht möglich, die AK-Aktivität mit einem individuellen Gerät zu bewerten.

Die Aufzeichnung und Verarbeitung von Pulswellen-Biosignalen ist heute in Herzdiagnosesystemen zur Überwachung der Herzfrequenz und der hämodynamischen Vorgänge in der menschlichen Arterienvene weit verbreitet. Eine der wirksamsten Methoden der Pulswellenregistrierung ist die Plethysmographie (PT). Eine evolutionäre Weiterentwicklung der Photoplethysmographie ist die Beschleunigungsplethysmographie (APG). Neben der Messung der Herzfrequenz ermöglicht die PGA auch die Schätzung der Phasencharakteristik der Pulswelle. Bewerten Sie mit der Berechnung ihrer gemeinsamen Merkmale wie SDNN, DEI, TP, Etc, EEI, DDI). Bewertung auf der Grundlage der Schätzung der Amplituden und der Dauer der einzelnen Wellen. Auch ihre Verhältnisse. Darüber hinaus werden die spektralen Komponenten der Herzfrequenzvariabilität durch die Berechnung der LF-, HF- und VLF-Spektren und ihrer Verhältnisse bewertet [12].

Bekannt ist die "Methode der Herzfrequenzdiagnostik", RF-Patent ¹2296501, Datum des Beginns der Patentgültigkeit: 09.03.2005, veröffentlicht: 10.04.2007, Bulletin ¹ 10.

Die Erfindung bezieht sich auf die Medizin und kann vor allem verwendet werden, um schnelle Informationen über den Zustand der Herztätigkeit zu erhalten. Die Methode besteht in der Durchführung der folgenden Vorgänge:
1) Anwendung eines Pulsumwandlungssensors an der radialen Arterie,
2) Umwandlung des Pulses in einen elektrischen Puls,
3) Einspeisung des Pulses in einen Autokorrelator mit Schätzung der Puls-Autokorrelationszeit,
4) Bestimmung der Amplituden- und Zeitcharakteristik der Pulswelle,
5) Zerlegung des Pulses in harmonische Komponenten mit Bestimmung der Amplitudenwerte jeder harmonischen Komponente,
6) Zerlegung des Pulses in harmonische Komponenten mit Bestimmung der Phasenwerte jeder harmonischen Komponente,
7) Berechnung des Verhältnisses der Amplitude der ersten harmonischen Komponente des Pulses zu den einzelnen Amplituden der harmonischen Komponenten,
8) Bestimmung des Verhältnisses der Anzahl der positiven Phasen der harmonischen Komponenten zur Anzahl der negativen Phasen der harmonischen Komponenten,
9) Bestimmung der Übereinstimmung mit dem Zustand der Herztätigkeit durch den gemessenen Wert der Verzögerungslinie in der Autokorrelation,
10) Bestimmung der Übereinstimmung mit dem Zustand der Herztätigkeit durch die Parameter des Amplituden-Zeit-Charakters entsprechend dem Verhältnis der Amplitude der ersten harmonischen Komponente des Impulses zu den einzelnen Amplituden der harmonischen Komponenten entsprechend dem Verhältnis der Anzahl der positiven Phasen der Oberschwingungsanteile zur Anzahl der negativen Phasen der Oberschwingungsanteile.

Die Erfindung erweitert den Anwendungsbereich des Pulsdiagnoseverfahrens. Sie ist jedoch nicht dazu gedacht, die AS-Aktivität zu bewerten. Es ist schwierig, sie für die individuelle AC-Bewertung in Form eines tragbaren, handlichen Geräts einzusetzen.

Nach dem RF-Patent Nr. 168518, Patentstartdatum: 13.04.2016, veröffentlicht: 07.02.2017, Bulletin Nr. 4 ist eine Vorrichtung zur Beschleunigungsphotoplethysmographie bekannt.

Das bekannte Gerät enthält einen Impulsgenerator, eine Lichtquelle, einen Fotodetektor, einen Strom-/Spannungswandler, einen Wechselspannungsverstärker, einen Synchrondetektor, einen Bandpassfilter, einen Analog-Digital-Wandler, einen Mikrocontroller, eine Doppeldifferenzierungseinheit, einen Maximaldetektor, einen Minimaldetektor und eine Teilungseinheit. Das Gerät ermöglicht es, einen Index des Funktionszustandes der arteriellen Gefäße einer Person zu bestimmen. Das Gerät ermöglicht weiter eine nicht-invasive Bewertung des Elastizitätsindexes der arteriellen Gefäße auf der Grundlage der Berechnung des Verhältnisses zwischen der Amplitude des ersten Minimums des beschleunigten Photoplethysmogrammsignals und der Amplitude des ersten Maximums des beschleunigten Photoplethysmogrammsignals.

Der Nachteil der Vorrichtung gemäß RF-Patent 168518 besteht darin, dass sie nur für die Bestimmung des Indikators für den Funktionszustand der menschlichen Arterien bestimmt ist. Das Gerät kann keine allgemeine nicht-invasive Bewertung des Körperzustands vornehmen.

Weiterhin ist eine Vorrichtung zur reflexiven Korrektur von Funktionsstörungen bekannt, RF-Patent Nr. 86870, Gültigkeitsdatum: 28. Mai 2009, veröffentlicht: 20.09.2009, Bulletin Nr. 26, als Prototyp. Das Gerät führt den Akabane-Test durch. Das Gerät dient der Überwachung und Korrektur des Funktionszustands des menschlichen Körpers. Die Überwachung basiert auf der Messung der Temperatur-Schmerzempfindlichkeitsschwellen (PSP) der Akupunkturpunkte (AP) am Eingang/Ausgang der Hauptkanäle. Das Gerät enthält das Diagnose- und Behandlungsmodul 1, eine Steuer- und Kommunikationseinheit 2 und einen Server 3, die durch bidirektionale Kommunikationskanäle 12 und 13 verbunden sind. Das Modul 1 ist als Stift 32 mit einer eigenständigen Stromversorgungseinheit 10 und einer Empfangs-/Sendeeinheit 11 ausgeführt. Das Modul 1 ist mit einem Element der thermischen Wirkung auf das AT in Form einer IR-LED 4 versehen. Die LED ist mit einem gesteuerten Stromgenerator 6, einer Emissionsanzeige 5, einem Start-/Stopp-Schalter 7, einer Ein-/Ausgangseinheit 8 und einem Mikrocontroller 9 verbunden. Ein Mobiltelefon oder ein Patientencomputer wird als Steuer- und Kommunikationseinheit 2 mit der Funktion des Empfangens/Sendens von Daten verwendet durch Anzeige der Daten und Steuerung des Moduls 1 und durch Eingabe eines residenten Programms. Der Handgriff 32 dient als peripherer Statussensor und kostengünstiges Expositionsgerät für den Patienten. Die gesamte Verarbeitung der adressierbaren individuellen Informationen jedes Patienten wird dem Server 3 anvertraut.

Der Akabane-Test erfordert jedoch besondere Kenntnisse. Der Test ist schwierig durchzuführen und zeitaufwändig. Bei der Bewertung der Schmerzempfindlichkeitsschwelle für Temperatur wird sie auch mit schmerzhaften Empfindungen in Verbindung gebracht. Dies schränkt die Nutzung durch Nicht-Experten auf diesem Gebiet ein.

Die Autoren haben festgestellt [1], dass die mit dem Akabane-Test gemessene AC-Aktivität eine hohe Korrelation mit verschiedenen elektrophysiologischen Parametern (Wenkenbach-Punkt (Wp)) aufweist und eine ARP-absolute und effektive Refraktärzeit (AvcERP) haben. Weitere Korrelationen bei der Wiederherstellung der Sinusknotenfunktion erforderliche Zeit - absolut -, für die Wiederherstellung der Sinusknotenfunktion erforderliche korrigierte Zeit (TSKFR) sowie die für die Wiederherstellung der Sinusknotenfunktion erforderliche korrigierte Zeit (CTSKFR) ) ermittelt. Diese korrelieren mit hämodynamischen Indizes (Auswurffraktion (EF), Kontraktilitätsrate des Herzmuskels (MCR), Größe des linken Vorhofs usw.) sowie der Herzfunktion. Auf diese Weise wurde ein grundlegender Zusammenhang festgestellt, der die Bildung der AK-Aktivitätsindikatoren ermöglicht hat, die aus einer Reihe von Leistungsindikatoren für das Herz gebildet sind. Die Indizes werden von der Pulswelle abgeleitet. Die Autoren führten zusätzliche Studien durch. Die Studien umfassten die Aufzeichnung von Fingerpulswellen mit Bewertung der Phasen- und Spektralcharakteristik von PTH und PGA. Dazu gehörten synchrone EKG-Aufzeichnungen, die mit den Daten des Akabane-Tests bei 325 Probanden verglichen wurden. Die Regressionsanalyse ergab, dass die bekannten Standard-PTH-Werte wie SDNN, DEI, TP, EI, ETC, EI, EEI, DDI usw. eine hohe Korrelation (/?<0,05) mit den durch den Akabane-Test gemessenen AC-Aktivitätswerten aufweisen. Dies gilt auch für eine Reihe zusätzlicher Zeitintervalle aus PGA und EKG, wenn diese synchron aufgezeichnet werden.

Diese Ergebnisse, die nur die signifikantesten Assoziationen der Kanäle (p<0,05) mit den PGA-Phasen- und Spektralindizes aufzeigen, sind in Tabelle 1 (Anhang 1) aufgeführt. Die folgenden Bezeichnungen der Hauptkanäle und Diagnosepunkte auf diesen Kanälen sind angegeben. In dieser Tabelle und in der vorliegenden Beschreibung der Erfindung werden folgende Punkte verwendet: LUI 1, Lunge; LI1, Dickdarm; NS9, Herzbeutel; TH1, Dreifacherwärmer; Ht9, Herz; SI1, Dünndarm; SP1, Milz; Livl, Leber; St45, Magen; GB44, Gallenblase; Ki 1, Niere; BI67, Harnblase. Der rechte und der linke Zweig (Seiten) des Kanals werden mit g bzw. 1 bezeichnet. Nachfolgend werden nur die Namen der ATs in der Beschreibung verwendet und die Punktnummern weggelassen.

DE 199 83 992 B4 offenbart die nicht-invasive Bestimmung von Blutbestandteilen in Bezug auf die Aktivität der Akupunkturkanäle. Die '992 offenbart, dass die energetische Aktivität dieser Kanäle durch Spektralanalyse des EKG oder der Pulswelle bestimmt werden kann. Die '992 offenbart auch die Stimulation mit IR-Strahlung bei gleichzeitiger Pulswellenmessung; alternativ werden Pulswellendaten verwendet, um die IR-Stimulation bis zum Eintritt von Schmerzen zu synchronisieren.

Ein technisches Problem, das derzeit besteht, ist der Mangel an Methoden und Geräten. Die Geräte sollen einfach zu bedienen sein und eine genaue individuelle Bewertung der Aktivität der Akupunkturkanäle ermöglichen. Die Bewertung hat auf der Grundlage des klassischen Akabane-Tests und der "Pulsdiagnose" zu erfolgen.

Die vorgeschlagene Erfindung zielt darauf ab, dieses technische Problem zu lösen, nämlich ein Modul, ein System und ein Verfahren zur individuellen Auswertung der Akupunkturkanalaktivität auf der Basis des klassischen Akabane-Tests und der "Pulsdiagnose" zu schaffen. Dies soll mit Hilfe der Analyse der Phasen- und Spektralkomponenten der Pulswelle und einer Reihe von EKG-Parametern erfolgen. Dadurch können individuelle Korrelationskoeffizienten auf derselben Messskala ermittelt werden. Dabei wird der Akabane-Test als allgemein anerkannte Referenzmethode zur Kalibrierung der Ergebnisse verwendet. Die Kombination dieser Techniken verbessert die Bewertungsgenauigkeit und erhöht den Nutzen des Systems insgesamt. Sie ermöglicht die Verwendung einer gemeinsamen metrischen Skala und gemeinsamer Regeln für die Bewertung von Testergebnissen.

Das technische Ergebnis, das mit dieser Methode, diesem System und diesem Modul erzielt wird, ist eine erhebliche Vereinfachung der Technik, da es keine kontinuierlichen aktiven Messungen der AK-Temperatur-Schmerzempfindlichkeitsschwellen nach dem Akabane-Test gibt. Dabei genügt es, den Finger auf den Pulssensor zu legen, um die AK-Aktivität nach einer Reihe von "teachable measures" zu bewerten. Bei Patienten mit Herz-Kreislauf-Problemen ist es auch möglich, ein EKG zu erhalten, indem die beiden Elektroden am Gehäuse des Geräts gleichzeitig mit den Fingern beider Hände getrennt berührt werden.

Das technische Ergebnis besteht weiter darin, dass die Effizienz der Nutzung von Computerressourcen erhöht wird. Sie besteht darin, die Kosten für das Gerät des Patienten zu senken und die Funktionalität des Geräts zu erweitern. Es ist zum Beispiel möglich, diese Methode, dieses System und dieses Modul auch für die Schätzung grundlegender Parameter der Hämodynamik durch Berechnung zu verwenden, zusätzlich zur AK-Aktivität.

Das technische Ergebnis kommt dadurch zustande, dass bei der Methode zur quantitativen Bewertung der Aktivität des Akupunkturkanals (AC) auf der Grundlage des Akabane-Thermopunktur-Tests vorgeschlagen wird, die Pulswelle des Probanden mittels Photoplethysmographie aufzuzeichnen. Dann können die Schwellenwerte seiner Temperaturschmerzempfindlichkeit synchron mit jeder Aufzeichnung an den Diagnosepunkten der Akupunkturkanäle während des Akabane- Thermopunkturtests gemessen werden. Die zweite beschleunigende Ableitung der primären Impulswelle ist zu bilden, die Standardwellen a, b, c, d, e, f sind zu extrahieren und deren Zeit- und Amplitudencharakteristiken sowie deren Beziehungen sind zu berechnen. Auf dieser Grundlage sind Standard-Phase Indizes der Pulswelle SDNN, DEI, TP, Etc, EEI, DDI sowie spektrale Komponenten der Herzfrequenzvariabilität mit Berechnung von LF-, HF-, VLF-Spektren und deren Verhältnissen zu berechnen. Für eine konkrete Person aus gepaarten Stichproben von Pulswellen- und Thermopunktur-Testindizes ist durch Methoden der mathematischen Analyse das Modell ihrer Wechselbeziehung mit der Bildung von Matrizen der Rückrechnung von Pulsindizes in AC-Aktivitätswerte zu schaffen. Weitere Quantifizierung.

AK-Aktivität allein auf der Grundlage von Pulswellenmessungen.

Weitere Unterschiede in der Methode sind:
- Bei Probanden mit kardiovaskulären Problemen werden zusätzlich EKG- Messungen durchgeführt. Es werden Zeitintervalle zwischen P-, Q-, R-EKG-Zähnen und der Welle auf dem beschleunigten Photoplethysmogramm gebildet. Diese Daten werden bei der Erstellung von Umrechnungsmatrizen von Puls- und EKG-Werten in AC-Aktivitätswerte in den Berechnungen weiter berücksichtigt.
- Die Pulswellen-Spektralindizes werden aus den R-Wellen des EKGs durch Cluster- und Faktorenanalyse bei der Verarbeitung großer Datenbanken mit gesammelten Statistiken zu verschiedenen Themen berechnet. Typische homogene Struktur- und Transformationskoeffizienten des Korrelationsmodells von Puls- und EKG-Indizes und AC-Aktivitätswerten werden identifiziert, um die Anzahl der Einzelproben bei Testmessungen zu reduzieren und die Effizienz der Methode zu erhöhen. Als Methoden der mathematischen Analyse werden Regressionsverfahren oder selbstlernende neuronale Netze eingesetzt.
- Die Koeffizienten der Bewertungsmatrizen können im Laufe der Zeit unter dem Einfluss verschiedener neuer externer und interner Faktoren auf den Organismus unzureichend werden. Um sie dynamisch an die Lebensbedingungen des Organismus anzupassen, werden in regelmäßigen Abständen (1 bis 3 Mal pro Woche) zusätzliche paarweise Messungen durchgeführt, um die Neuberechnungsmatrizen unter Berücksichtigung der neuen Daten zu korrigieren.
- Die Pulswelle wird von der Fingerendgliedsohle oder von den Weichteilen in einem Bereich wie dem Handgelenk aufgezeichnet.

Das technische Ergebnis wird auch dadurch erreicht, dass im System zur quantitativen Bewertung der Aktivität der Akupunkturkanäle (AK), das auf dem Akabane-Thermopunktur-Test basiert, das Modul zur Datenerfassung und -verarbeitung mit der Möglichkeit der Aufzeichnung der Parameter der Pulswelle ausgestattet ist. Das System zur quantitativen Bewertung umfasst ein Modul zur Datenerfassung und - verarbeitung. Das Modul ist so implementiert, dass es die Schwellenwerte der Temperaturschmerzempfindlichkeit (PPS) von Akupunkturpunkten (AP) des AC-Eingangs/-Ausgangs messen kann. Das System besteht aus einem Server und einem Steuerungs- und Kommunikationsmodul. Dieses ist über bidirektionale Datenkommunikationskanäle mit dem Datenerfassungs- und Verarbeitungsmodul und dem Server verbunden. Der Server ist so konzipiert, dass er ein Korrelationsmodell zwischen den Parametern der empfangenen Impulswellenform und den Werten der Schwellenwerte der Temperaturschmerzempfindlichkeit der AK durch den Akabane-Test erstellt. Dabei besteht die Möglichkeit, Umrechnungsmatrizen von den Pulswellenwerten zu den Werten der AK-Aktivität zu erstellen. Das Steuerungs- und Kommunikationsmodul ist so implementiert, dass es die Funktion des Empfangens/Sendens von Daten vom Server und dem Datenerfassungs- und - verarbeitungsmodul ausführen kann. Weiterhin kann es die Verarbeitung, Anzeige von Informationen und Steuerung des Datenerfassungs- und Verarbeitungsmoduls und der Datenverarbeitung auf dem Server ausführen.

Das System verfügt über die folgenden zusätzlichen Funktionen:
- Das Modul zur Datenerfassung und -verarbeitung ist mit der zusätzlichen Möglichkeit der Aufzeichnung von EKG-Parametern ausgestattet. In diesem Fall hat der Server die Möglichkeit der Schaffung eines Modells der zusätzlichen Korrelation zwischen der erhaltenen Pulswelle, EKG-Parameter und Werten der Temperaturschwellen der AC-Schmerzempfindlichkeit durch den Akabane-Test mit Erstellung von Umrechnungsmatrizen von Puls- und EKG-Werten in AC-Aktivitätswerte.
- Die Datenerfassungs- und Verarbeitungseinheit enthält 2 Fingerelektroden und eine EKG-Aufzeichnungseinheit.
- Der optionale Pulswellensensor ist als eigenständiges Modul konzipiert. Das Modul kommuniziert in beide Richtungen mit dem Steuerungs- und Kommunikationsmodul. Es kann z. B. in ein Uhren- oder Armbanddesign mit einem System zur Übertragung des primären Pulswellenmusters an das Steuer- und Kommunikationsmodul integriert werden.
- Ein Mobiltelefon oder Tablet oder ein Personalcomputer wird als Steuerungs- und Kommunikationsmodul verwendet.
- Das Modul zur Datenerfassung und -verarbeitung ist als eigenständiges Gerät konzipiert. Auf der Oberfläche des Geräts befinden sich ein IR-Sender zur Schätzung der Empfindlichkeitsschwellen nach dem Akabana-Test, ein Pulswellensensor und ein Bildschirm. Weiter sind eine USB-Buchse zum Aufladen des Akkus und zusätzlich 2 Elektroden zur EKG-Aufzeichnung angebracht.
- Das Erfassungs- und Verarbeitungsmodul enthält zusätzlich 2 EKG-Elektroden.

Das technische Ergebnis wird auch dadurch erreicht, dass ein Bildschirm zur Anzeige von grafischen Informationen und ein Pulswellensensor in das Grundmodul zur Datenerfassung und -verarbeitung integriert werden.

Der Sensorausgang ist in Reihe mit dem Stromwandler, dem Verstärker, dem Synchrondetektor und dem Bandpassfilter geschaltet. Der Ausgang des Verstärkers und der Ausgang des Bandpassfilters sind mit dem Mikrocontroller verbunden. Der erste Kontakt des Start/Stopp-Schalters des Pulswellensensors ist mit dem gemeinsamen Massebus verbunden. Der zweite Kontakt ist mit dem Eingang/Ausgang des Mikrocontrollers verbunden. Das Basismodul zur Datenerfassung und -verarbeitung enthält ein thermisches Akupunkturpunktelement (AP) in Form einer Infrarot-LED. Die LED ist über einen gesteuerten Stromgenerator an eine Stromversorgung mit einem Start-/Stopp-Schalter angeschlossen. Der Stromgenerator ist über einen Mikrocontroller mit der Sende-/Empfangseinheit verbunden. Dieses Gerät ist für den Anschluss an das Steuerungs- und Kommunikationsmodul vorgesehen. Die Kontakte des Start/Stopp-Schalters der IR-LED des thermischen Effekts sind mit dem Mikrocontroller verbunden. Dieser sowie der Stromgenerator, die LED-Kathode, der IR-LED-Wärmeeffekt-Start/Stopp-Schalter und die Sende-/Empfangseinheit sind mit einer gemeinsamen Erdungsschiene verbunden.

Weitere Unterschiede im vorgeschlagenen Modul sind folgende:
- Es enthält 2 EKG-Elektroden und eine EKG-Aufzeichnungseinheit. Diese sind mit dem Mikrocontroller und der Stromversorgung verbunden.
- Der Pulswellensensor ist als eigenständiges Modul konzipiert. Das Modul kommuniziert in beide Richtungen mit dem Steuerungs- und Kommunikationsmodul, ist mit einem Server verbunden und z. B. in eine Uhr oder ein Armband integriert. Entwurf eines Verarbeitungssystems zum Empfang und zur Übertragung eines Diagramms der primären Pulswelle oder PGA-Welle mit den Werten der Wellenindikatoren a, b, c, d, e, f an das Steuer- und Kommunikationsmodul.
- Das Modul zur Datenerfassung und -verarbeitung ist als eigenständiges Gerät konzipiert. Auf der Oberfläche des Geräts befinden sich ein IR-Sender zur Schätzung der Empfindlichkeitsschwellen nach dem Akabane-Test, ein Pulswellensensor und ein Bildschirm. Eine USB-Buchse zum Aufladen des Akkus und zusätzlich 2 Elektroden zur EKG-Aufzeichnung sind angebracht.
- Das Datenerfassungs- und Verarbeitungsmodul enthält zusätzlich 2 EKG-Elektroden.

### Das Wesentliche der Erfindung

Die vorgeschlagene Erfindung wird anhand des beigefügten Bildmaterials näher erläutert. Es zeigen:

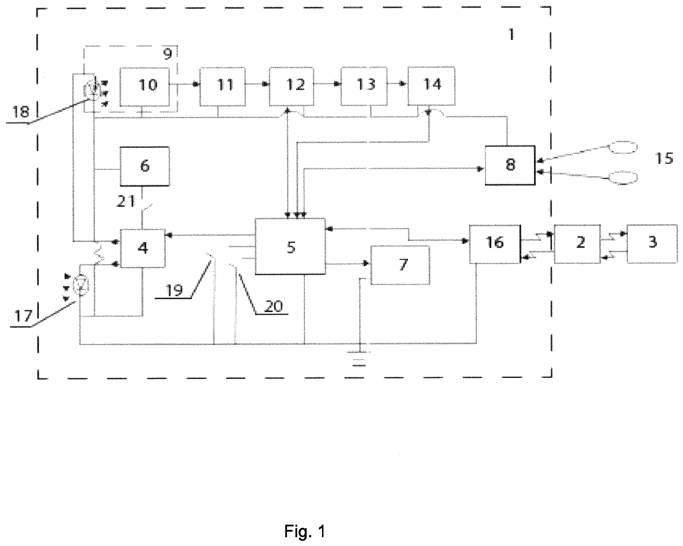

- Fig. 1: eine Variante des Strukturdiagramms des vorgeschlagenen Systems (mit EKG-Gerät und Elektroden),

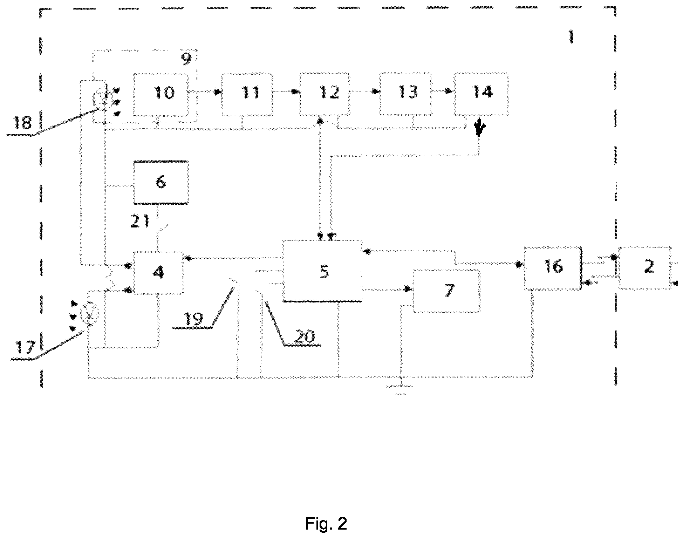

- Fig. 2: eine Variante des Strukturschemas des Systems ohne Elektroden und EKG-Gerät,

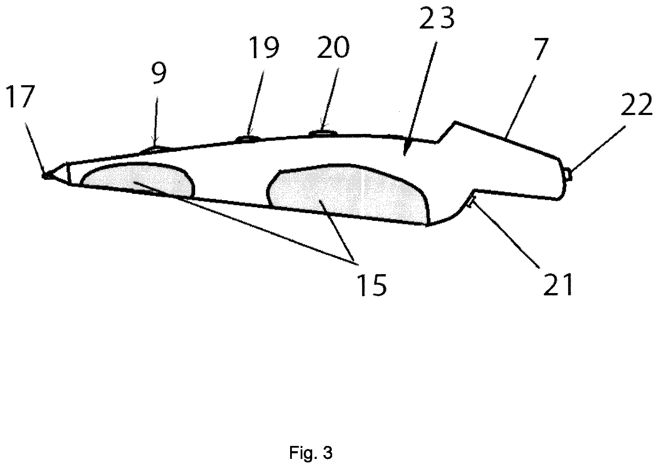

- Fig. 3: das Modul zur Erfassung und Verarbeitung von Informationen und
- Fig. 4: ein Diagramm der primären Pulswellenaufzeichnung, die mit der Photoplethysmographie (PTG) gewonnen wurde und ein Diagramm der zweiten mathematischen Ableitung, erhalten durch beschleunigte Plethysmographie (APG) mit den Wellen a, b, c, d, e, f. und EKG-Aufzeichnung mit P-, Q-, R-, S-Wellen und Zeitintervallen zwischen ihnen.
In den Zeichnungen werden die folgenden Symbole verwendet:
1. Modul zur Datenerfassung und -verarbeitung
2. Steuerungs- und Kommunikationsmodul
3. Server
4. Stromerzeuger
5. Mikrocontroller
6. Stromversorgungseinheit
7. Bildschirm
8. EKG-Gerät
9. Impulswellen-Sensor
10. Foto-Empfänger
11. Signalwandler
12. Verstärker
13. Synchrondetektor
14. Bandpassfilter
15. Elektroden des EKG-Geräts
16. Empfangs-/Sendeeinheit
17. LED-Infrarot-Element zur Wärmebehandlung von Akupunkturpunkten
18. IR-LED-Beleuchtung des Pulswellensensors
19. Start/Stopp-Kippschalter für Pulswellenaufzeichnung
20. Start/Stopp-Schalter für das Akupunkturelement
21. Start/Stopp-Schalter für die Stromversorgung
22. USB-Anschluss zum Aufladen der Batterien
23. Gehäuse für Datenerfassungs- und Verarbeitungsmodule

Der Autor stellte fest, dass das AK-System die einzelnen menschlichen Organe und physiologischen Systeme in ein einheitliches Ganzes integriert. In diesem Fall ist ein bestimmter Herzleistungsindikator nicht mit einem einzigen Kanal verbunden, sondern mit mehreren. Um die Werte einer bestimmten AK auf individueller Ebene zu bestimmen, werden daher die zuverlässigsten Verbindungen mehrerer Herzleistungsindikatoren ermittelt. Dies geschieht durch die Methode der schrittweisen Regression. Die Korrelationen spiegeln sich in den Pulswellen- und EKG-Indizes wider, identifiziert, um das effektivste Modell für die Berechnungen zu erhalten.

Neben der Regressionsanalyse eignet sich auch die Methode des "selbstlernenden neuronalen Netzes" für diesen Zweck.

Die Energieaktivität des Akupunkturkanals kann mit dem Akabane-Test beurteilt werden. Zu diesem Zweck werden thermische Impulse von Infrarotstrahlung (IR-Strahlung) an den "Input-Output"-Punkt des Kanals gesendet (Wellenlänge 780 - 1400 nm, Frequenz 1 Hz, Rechteckpulse mit einem Tastverhältnis **von %** und einer Strahlungsleistungsdichte von 50 - 400 mW/cm². Der Wert der Energieaktivität des Kanals kann anhand der Anzahl der Pulse beurteilt werden, die vom Beginn der Exposition bis zum Auftreten der ersten Schmerzempfindungen emittiert wurden [1 - 7]. Zur Bewertung der AK-Aktivität durch Pulsdiagnose wird ein System individueller Berechnungen auf der Grundlage von "Lehrmessungen" während einer Reihe von Statistiken verwendet. Zu diesem Zweck wird die Pulswelle (in einigen Versionen auch das EKG) zunächst 2 - 5 Minuten lang aufgezeichnet, und die wichtigsten Indizes werden berechnet. Anschließend wird der Akabane-Test durchgeführt, und die Ergebnisse werden an den Server gesendet. Es sollte mindestens 10 - 15 solcher gepaarten Einzelaufnahmen geben. Sie werden durch Korrelations- und Regressionsanalysen auf dem Server verarbeitet, mit Berechnung der Korrelationskoeffizienten zwischen den einzelnen Indizes der BPH (Beschleunigungsplethysmographie) und der zugehörigen AC. Das Ergebnis ist eine Matrix mit einzelnen Umrechnungskoeffizienten. Die Genauigkeit der Berechnung kann mittels Durchführung von Akabane-Kontrolltests durch den Vergleich der berechneten PGA-Daten mit den klassischen Daten überprüft werden. Anschließend werden 2 Tabellen mit der Herzfrequenz und den Ergebnissen des Akabane-Tests zum Vergleich auf dem Smartphone-Bildschirm angezeigt. Im Allgemeinen gilt: Je mehr Trainingsbeobachtungen, desto genauer die Berechnung der Herzfrequenz.

Zur Schätzung der Spektralindizes mit Hilfe der FFT-Transformation durch PGA über eine Stichprobe von 3 - 5 Minuten werden die Spektralindizes im LF-, VLF- und HF-Band benutzt. Nach den Daten der Korrelationsanalyse (Tabelle 1, Anhang 1) können auf diese Weise hauptsächlich die Kanäle LU, PC, TE, SP, ST und KI geschätzt werden. Entsprechend der Dynamik der Veränderungen der Herzrhythmusvariabilität mit Schätzung der Zeitintervalle zwischen den Scheitelwellen und mit Schätzung der mittleren Abweichung in ms kann der SDNN-Indikator berechnet werden. Der Index weist hohe Korrelationskoeffizienten mit den Kanälen LU, PC, SI, ST, KI, HT und SP auf.

Der TP-Index charakterisiert den Zustand des autonomen Nervensystems und weist signifikante Korrelationen mit den Kanälen LU, TE, SI, ST, KI auf. ETc spiegelt die Auswurfzeit wider und korreliert vor allem mit den Kanälen LU, LI, TE, HT, SI, SP, LR, ST, GB, KI und BL. Die Berechnung desselben AC für mehrere Parameter durch Mittelwertbildung verbessert ebenfalls die Genauigkeit und Stabilität der Schätzung. Durch Vergleich von PGA-Wellen und EKG werden die Zeitintervalle P-a, Q-a, R-a ermittelt. S-a. Die Intervalle verbessern die Qualität der Berechnungsmodelle für verschiedene Herzerkrankungen erheblich.

**Tabelle 1 :**

| |
|---|
| AC-Säule - Markierung der Akupunkturkanäle; |
| d - der rechte Zweig der Kanäle; |
| 1 - linker Zweig der Kanäle; |
| P - Signifikanz der Korrelation bei p kleiner als 0,05 (Fehler kleiner als 5%); |
| g - der Korrelationskoeffizient; |
| Anzahl der Beobachtungen - 325 Personen. |

Im Gegensatz zu Korrelationsbeziehungen ermöglicht die Regression die Konstruktion effizienter, komplexer Berechnungsmodelle. Dies kann durch die Einbeziehung von Indikatoren mit geringen Korrelationswerten erreicht werden. Durch die Verwendung mehrerer solcher Indikatoren für die Berechnungen wird die Genauigkeit der Schätzung jedoch noch erheblich verbessert.

Tabelle 1 (Anhang 1) zeigt, dass nicht alle Programme mit diesen Indikatoren hoch korreliert sind. In diesem Fall werden für die Berechnungen Regressionsmodelle der Beziehungen zwischen den Kanälen verwendet. Da alle 12 AKs im System der 5 Primärelemente starr miteinander korreliert sind, spiegeln sich Veränderungen in der Aktivität von Kanal 1 in allen anderen Kanälen wider [5].

Wenn wir also die Aktivität von z.B. 3-4 Kanälen mit hohen Korrelationskoeffizienten mit APT-Indizes kennen, können wir mit Hilfe eines Regressionsmodells der Beziehungen zwischen den Kanälen, an denen sie beteiligt sind, die Werte mehrerer anderer ACs mit geringerer Zuverlässigkeit der Abhängigkeiten von den angegebenen Indizes berechnen. Dieser Ansatz ermöglicht auch eine weitere Verbesserung der Genauigkeit der AA-Schätzungen durch diese Methode.

Neben den Regressionsmodellen können auch selbstlernende neuronale Netze eingesetzt werden. Dadurch wird auch die Genauigkeit der Schätzung verbessert, da die Beziehungen zwischen allen AKs berücksichtigt werden.

Die vorgeschlagene Methode kann mit Hilfe eines Systems zur Quantifizierung der Akupunkturkanalaktivität umgesetzt werden. Dieses System enthält das Datenerfassungs- und Verarbeitungsmodul 1. Das Modul dient zur Messung der Schwellenwerte für die Temperaturschmerzempfindlichkeit (PPS) von Akupunkturpunkten (APs) des AC-Eingangs/-Ausgangs. Es enthält einen Server 3 und ein Steuer- und Kommunikationsmodul 2, das über bidirektionale Datenkommunikationskanäle mit dem Datenerfassungs- und -verarbeitungsmodul 1 und mit dem Server 3 verbunden ist. Das Datenerfassungs- und -verarbeitungsmodul 1 dient der Aufzeichnung von Pulswellenparametern. Der Server 3 ist so konzipiert, dass er ein Modell der Beziehung zwischen den empfangenen Pulswellenparametern und den Werten der AC-Temperatur-Schmerzempfindlichkeitsschwellen gemäß dem Akabane-Test erstellen kann, durchgeführt mit der Erstellung von Umrechnungsmatrizen von den Pulswellenwerten zu den Werten der AK-Aktivität. Das Steuer- und Kommunikationsmodul 2 ist in der Lage, Daten vom Server 3 und dem Datenerfassungs- und - verarbeitungsmodul 1 zu empfangen und zu übertragen.

Das Datenerfassungs- und -verarbeitungsmodul 1 enthält in der Grundversion ein Element zur thermischen Beeinflussung der Akupunkturpunkte (AP) in Form einer Infrarot-IR-Lichtdiode 17. Über einen gesteuerten Stromgenerator 4 ist es mit einem Start-/Stopp-Schalter 21 an die Stromversorgungseinheit 6 angeschlossen. Diese ist über den Mikrocontroller 5 mit der Empfangs-/Sendeeinheit 16 verbunden. Das Gerät ist für den Anschluss an das Steuerungs- und Kommunikationsmodul 2 vorgesehen. Gleichzeitig sind die Kontakte des Start/Stop-Schalters 20 der IR-Wärme-LED mit dem Mikrocontroller 5 verbunden, die wie der Stromgenerator 4, die Kathode der LED 17, der Thermoeffekt-IR-LED-Start/Stopp-Schalter 20 und die Empfangs-/Sendeeinheit 16 mit einer gemeinsamen Erdungsschiene verbunden sind. Das Modul umfasst auch einen Bildschirm 7 zur Anzeige von grafischen Informationen, einschließlich Pulswellensensor 9. Der Sensorausgang ist in Reihe mit dem Stromwandler 11, dem Verstärker 12, dem Synchrondetektor 13 und dem Bandpassfilter 14 geschaltet. Der Ausgang des Verstärkers und der Ausgang des Bandpassfilters sind mit dem Mikrocontroller 5 verbunden. Der erste Kontakt des Start/Stopp-Schalters 19 des Pulswellensensors 9 ist mit der gemeinsamen Erdungsschiene verbunden. Der zweite Kontakt ist mit dem Eingang/Ausgang des Mikrocontrollers 5 verbunden. Zusätzlich kann das Datenerfassungs- und -verarbeitungsmodul 2 EKG-Aufzeichnungselektroden 15 und eine EKG-Aufzeichnungseinheit 8 enthalten, die mit dem Mikrocontroller 5 und dem Netzteil 6 verbunden ist.

Der Pulswellensensor 9 kann eine steuerbare IR-LED 18 zur Beleuchtung enthalten. Die LED ist optisch mit dem Fotodetektor 10 verbunden. Dabei wird ein zusätzlicher Ausgang des Stromgenerators 4 mit der Anode der Infrarot-Beleuchtungs-LED 18 verbunden. Die Kathode des Generators ist mit einer gemeinsamen Masseschiene verbunden.

Der Pulswellensensor 9 kann auch als eigenständiges Modul ausgeführt werden. Das Modul kommuniziert in beide Richtungen mit dem Steuer- und Kommunikationsmodul 2, dem Server 3. Das Modul wird z. B. in eine Uhr oder ein Armband mit einem Verarbeitungssystem integriert, um eine erste Pulswellenform zu erhalten. Das Plethysmogramm wird an das Kontroll- und Kommunikationsmodul übertragen, um dort die PGA-Wellenformkurve mit der Berechnung der Wellenindikatoren a, b, c, d, e, f zu erhalten.

Modul 1 ist als eigenständiges Modul konzipiert. Auf seiner Oberfläche 23 befindet sich ein Pulswellensensor 9. Der Sensor besteht aus einem IR-LED-Strahler 18 und einem Fotodetektor 10. Es umfasst außerdem einen USB-Anschluss 22 zum Aufladen der Batterien, die Kippschalter 19, 20 und 21 sowie die Elektroden 15 des EKG-Geräts. Es enthält auch eine IR-LED 17 für thermische Wirkung auf Akupunkturpunkte.

Das Mobiltelefon oder der Computer des Benutzers wird als Steuer- und Kommunikationseinheit 2 verwendet. Mit seinem internen Mikroprozessor, der Anzeige, den Bedienelementen und den Empfangs- und Sendeeinheiten werden die Funktionen der PGA-Konstruktion auf der Grundlage der zweiten Beschleunigungsableitung, der Extraktion der Pulswellen-Phasenkomponente, der Datenanzeige und der Steuerung von Modul 1 übernommen. Serverteil 3 dient dazu, individuelle Berechnungsmodelle zu erstellen.

Das Steuermodul 2 extrahiert per Software die primäre Pulswelle und ihre zweite Beschleunigungsableitung als b-, c-, d- und e-Wellen. Aus dieser Ableitung werden die Standard-Pulswellenphasenindizes (SDNN, DEI, TP, Etc, EEI, DDI) berechnet. Sie werden auf der Grundlage der geschätzten Wellendauern und ihrer Verhältnisse berechnet. Es gibt anfänglich positive (a), früh negative (b), wieder ansteigende (c), spät wieder abfallende (d) und diastolische positive (e) diastolische positive (f) diastolische negative. Aus diesen Determinanten lassen sich die folgenden Parameter berechnen: b/a-Verhältnis, c/a-Verhältnis, d/a-Verhältnis, e/a-Verhältnis. Auf der Grundlage ihrer Verhältnisse werden zusätzliche Parameter für den Satz von Statistiken berechnet. Die Methode zur Extraktion dieser Wellen ist ausführlich beschrieben [8 - 9].

Darüber hinaus werden bei der EKG-Auswertung zusätzliche P-a-, Q-a-, R-a- und S-a-Intervalle ausgewertet. Die spektralen Komponenten der Herzrhythmusvariabilität werden durch die Berechnung der LF-, HF- und VLF-Spektren und ihrer Verhältnisse bewertet. Die Analyse von P-a, Q-a, R-a, S-a ist besonders bei Erregungsleitungsstörungen des Herzens wirksam. Die Analyse von P-a, Q-a, R-a, S-a ist besonders effektiv bei kardialen Erregungsleitungsanomalien.

Zur Umsetzung dieser Funktionen wird eine spezielle Software installiert.

Die EKG-Aufzeichnungseinheit 8 kann z. B. auf einer integrierten Mikroprozessoreinheit AD8232 basieren. Dieses Gerät ermöglicht die Extraktion, Verstärkung und Filterung von kleinen Biosignalen in Anwesenheit von Rauschen während der Aufzeichnung oder eines ähnlichen Mikrochips MAH30003.

Die Funktionsweise des vorgeschlagenen Systems ist wie folgt.

Das Datenerfassungs- und Verarbeitungsmodul 1 lädt das Netzteil 6 über die Buchse 22 aus dem externen Netzteil.

Der Schalter 21 versorgt das Modul mit Strom von der Stromversorgungseinheit 6. Dadurch wird die Bluetooth-Kommunikationssoftware mit dem Steuermodul 2 gestartet. In der folgenden Beschreibung wird davon ausgegangen, dass das Mobiltelefon des Patienten als Modul 2 verwendet wird. Wenn die Verbindung hergestellt ist, wird der Bildschirm des Geräts eingeschaltet. Dieser zeigt den Stand der Kommunikation mit dem Smartphone und den Ladezustand des Akkus an. Das Hauptmenü wird auch auf dem Bildschirm des Mobiltelefons angezeigt. Das Menü bietet folgende Optionen: "Bewertung des Kanalstatus durch Akabane-Test", Bewertung des Kanalstatus nur durch Pulswellen und zusammen mit dem EKG, "Daten an den Server senden" und "Daten vom Server empfangen". Im Telefon wird das Programm durch die Auswahl "Akabane-Test" aktiviert. Das Programm ermöglicht es, die Aktivität der ACs auf dem IF zu messen. Auf dem Smartphone-Display wird abwechselnd ein Bild des Standorts der AKs angezeigt, beginnend mit der ersten. Die Person, die die Messung durchführt, nimmt die Messung mit Hilfe der Aufforderungen und Punktbilder zur Bewertung der AKs selbständig vor. Der IR-Strahler des Geräts mit IR-LED 17 ist zur Erwärmung auf dem ersten Punkt der gewählten AK mit direktem Kontakt der LED 17 mit der Hautoberfläche zu platzieren und der Start/Stopp-Schalter 20 zu drücken. Die Taste schaltet den gesteuerten Stromgenerator 6 in den Messmodus und aktiviert den Timer/Counter des Mikrocontrollers 5. Die IR-Impulsstärke kann im Menü auf dem Smartphone-Bildschirm geändert werden.

Typischerweise wird in der Betriebsart "ZF-Auswertung" eine Impulsfolge mit einer Wiederholperiode von 1 sec. von einem gesteuerten Stromgenerator 4 z. B. an die IR-Diode angelegt. Die Pulsdauer liegt innerhalb von 0,75 Sekunden bei einem Tastverhältnis von 3/4 und einem Stromwert, der IR-Strahlung mit einer Leistungsdichte im Puls von 200 mW/cm² liefert. Die Anzahl der Impulse wird gezählt, bis der Schwellenwert der Temperaturempfindlichkeit für jeden Punkt erreicht ist. Eine andere Variante der Durchführung des Tests ist eine kontinuierliche Erwärmung in einem nicht gepulsten Modus mit Zählung der Belichtungszeit in Sekunden. Die Wärmequelle ist eine IR-LED 17. Die Linse der LED ist eine Halbkugel mit einem Radius von etwa 1,5 mm. Dadurch kann die AT-ZF von der Hautoberfläche aus gemessen werden, die flächenmäßig dem morphologischen AT-Substrat entspricht.

Die abgestrahlte Wellenlänge kann im Bereich von 780 bis 1600 nm liegen. Das liegt im "Wasserdurchlässigkeitsfenster" und der optimalen Haut- und Gewebedurchlässigkeit (siehe z.B. Patent RU N2122208, MPK 6 G01N 33/49, A61B 5/00, op. 20.11.1998). Vorzugsweise wird als LED 17 eine IR-LED des Typs ZAL107B mit einer Emissionswellenlänge von 920 nm verwendet. Diese Art von LED hat zuverlässige Ergebnisse gezeigt. Die Leistungsdichte wird unter der Bedingung gewählt, dass ein dynamischer Bereich der Änderung der ZF der Kanäle gewährleistet ist. Die Kanäle befinden sich in verschiedenen Zuständen, die von 2 bis 100 Sekunden reichen, mit einem durchschnittlichen Schwellenwert von 5 bis 10 Sekunden. Diese Bedingung wird durch eine IR-Leistungsdichte von 200 mW/cm2 im Puls erfüllt.

Der gepulste Modus mit einer Pulsdauer von 1 s und einem Tastverhältnis von % (0,75 s Puls und 0,25 s Pause) wurde empirisch gewählt, ausgewählt auf der Grundlage von Mehrfachbeobachtungen, und gibt eine deutlichere Schwelle der Schmerzwahrnehmung (PP) an.

Wenn der Patient das erste Schmerzempfinden verspürt, wird die Taste 20 ein zweites Mal gedrückt. Der daraus resultierende Tabellen-Strobe/Impuls schaltet den gesteuerten Stromgenerator 4 ab und stoppt den Zeitgeber/Zähler im Prozessor 5, das Zeitintervall in Sekunden oder die Anzahl der 1Hz-Impulse vom Beginn der AT-Bestrahlung bis zum Auftreten der ersten Schmerzempfindung. Das Intervall wird vom Timer/Zähler gezählt und entspricht der physiologischen Schwelle der Temperaturschmerzempfindlichkeit (PPS) des jeweiligen Kanals.

Ist die Impulsleistung bekannt, kann aus der aufgewendeten Energiemenge die Zwischenfrequenz in Joule berechnet werden. Die Anzahl der Impulse während eines Tests wird auf dem Bildschirm des Geräts und auf dem Smartphone angezeigt. Dies wird mit einer zusammenfassenden Tabelle auf dem Smartphone der IF-Verteilung der getesteten AKs angezeigt. Diese Messdaten werden dann an Server 3 gesendet. Auf dem Server wird der Patient identifiziert, zum Beispiel auf der Grundlage einer Telefon-SIM-Karte oder einer eindeutigen Autorisierungs-ID.

In diesem Fall stützt sich die Bewertung der Kanalaktivität nach dem Akabane-Test auf die Bewertung der Zwischenfrequenz nach bekannten Verfahren (1 - 5).

Für die pulsgestützte AK-Aktivitätsschätzung wird nach dem Drücken der Taste 19 der Stromgenerator 4 gestartet. Der Generator sorgt für die Beleuchtung der IR-Diode 18 im Optokoppler des Impulsgebers 9. Der Optokoppler wird von der Testperson mit der Fingernagelkuppe des Zeigefingers der linken oder rechten Hand berührt. Die optische Durchlässigkeit des Gewebes ändert sich in Abhängigkeit von der Pulsfüllung. Infolgedessen werden unterschiedliche Beleuchtungsstärken im Fotodetektor 10 erfasst. So kann bei der automatischen Anpassung jedes Mal der optimale Aufzeichnungsmodus gewählt werden. Dieses Signal wird dem Eingang des Stromwandlers 11 zugeführt, dessen Ausgang mit dem Eingang des Verstärkers 12 verbunden ist. Der Verstärker wird durch den Mikroprozessor 5 gesteuert durch Regelung der IR-LED-Emissionsstufe 17 durch die Einheit 4 bis zum Erreichen der für die weitere Verarbeitung erforderlichen Pulswellenamplitude. Diese Welle wird von dem Synchrondetektor 13 erfasst. Der Eingang des Detektors ist mit dem Ausgang des Verstärkers 12 verbunden, und der Ausgang des Synchrondetektors 13 ist mit dem Bandpassfilter 14 verbunden. Der Filter reinigt das Signal von Fremdlichteinflüssen und Chattering. Der Filter ist wiederum mit dem Eingang des Mikrocontrollers 5 verbunden. Der Ausgang des Mikrocontrollers 5 ist mit dem Eingang der Sende-/Empfangseinheit 16 verbunden. Das Funksignal von diesem Block wird an das Smartphone übertragen. Das Smartphone führt seine Phasen- und Spektralverarbeitung auf Software-Ebene durch, was große Rechenressourcen erfordert.

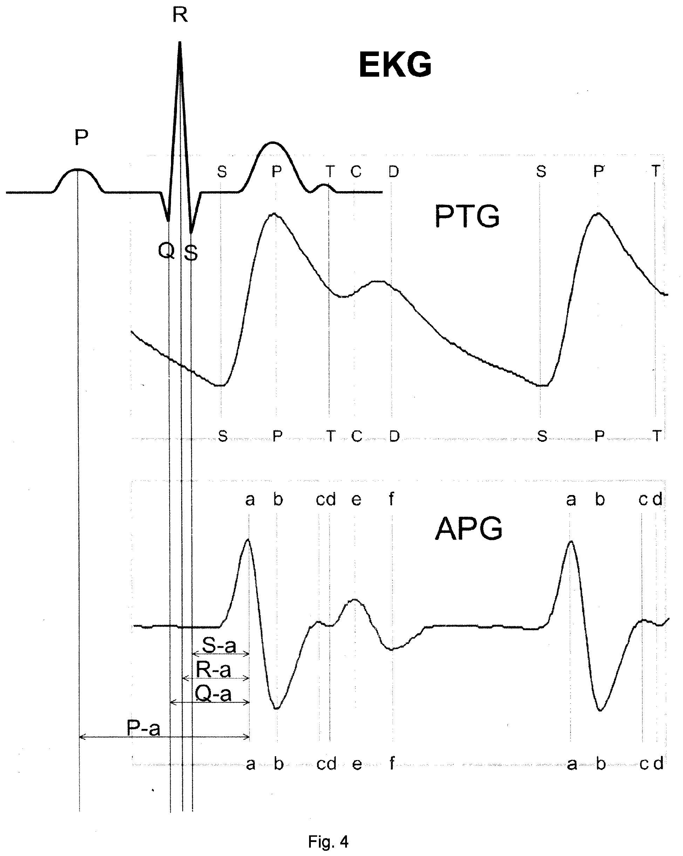

Zunächst wird die zweite Beschleunigungsableitung des RTG im Smartphone extrahiert (Fig. 4). Die Zeit- und Amplitudeneigenschaften der Wellen a, b, c, d, e, f werden dann berechnet. Wenn der EKG-Block 8 auf Befehl des Smartphones aktiviert wird, werden darin zusätzlich die Zeitintervalle P-a, Q-a, R-a, S-a (Fig. 4) durch Vergleich mit PGA-Wellen überwacht. Diese Daten werden über den Funkkanal des Smartphones an den Server in eine individuelle Datenbank gesendet. Die visuelle Bewertung der Qualität der Registrierung der primären Pulswelle PTG erfolgt auf dem Bildschirm 7 des Geräts. Der Geräteeingang ist mit dem Ausgang 5 des Mikrocontrollers verbunden. Damit kann die Qualität des gegen den Pulssensor gedrückten Fingers und die Qualität der EKG-Aufzeichnung überwacht werden, wenn diese gleichzeitig aufgezeichnet wird. Zusätzlich wird die Beschleunigungswellenextraktion mit ihren a-f-Peaks und EKG P-S-Peaks als gemitteltes Bild mehrerer Pulswellenaufzeichnungen (z. B. 5 - 10 Wellen werden verarbeitet und gemittelt) auf dem Smartphone-Bildschirm kontrolliert. Dadurch wird auch die Genauigkeit der Messungen erhöht.

Die IF-Kanal- und Pulswellenmessdaten jedes Patienten werden auf Server 3 verarbeitet.

Zur Bewertung der AK-Aktivität durch Pulsdiagnose wird ein System von Einzelberechnungen auf der Grundlage von "Lehrmessungen" im Rahmen einer Reihe von Statistiken verwendet. Zu diesem Zweck wird zunächst eine Pulswelle, oder in Kombination mit dem EKG, 2 - 5 Minuten lang aufgezeichnet und deren grundlegende Phasen- und Spektralindizes berechnet. Danach wird der Akabane-Test durchgeführt, und die Messergebnisse werden an Server 3 gesendet. Es sollten mindestens 10 - 15 solcher gepaarten Einzelaufnahmen vorhanden sein, um effektive Modelle zu erstellen. Die erhaltenen Indikatoren werden durch Korrelations- und Regressionsanalyse auf dem Server 3 mit Berechnung von Korrelationskoeffizienten zwischen einzelnen ARG-Indikatoren und den zugehörigen ACs verarbeitet. Das Ergebnis ist eine Matrix mit individuellen Umrechnungsfaktoren. Die Genauigkeit der Berechnung kann durch die Durchführung von Akabane-Kontrolltests durch den Vergleich der berechneten Daten mit den klassischen Daten überprüft werden. Eine zweite Tabelle mit der Herzfrequenz und den Ergebnissen des Akabane-Tests wird dann zum Vergleich auf dem Smartphone-Bildschirm angezeigt. Im Allgemeinen gilt: Je mehr "Lernbeobachtungen", desto genauer sind die Herzfrequenz- und EKG-Berechnungen.

Mit einem zusätzlichen Gerät in Form eines Armbands kann die AK-Aktivität jederzeit überwacht werden, mit einem Herzfrequenzsensor oder einem geeigneten Fitness-Tracker mit Herzfrequenzaufzeichnung. Das Gerät besteht aus einer Reihe von Modul-1-Einheiten mit Datenübertragung über Bluetooth auf das Smartphone. Das Gerät kann nach den oben beschriebenen Prinzipien arbeiten. Die Übertragung und Verarbeitung von Daten aus einem solchen Gerät kann diskret erfolgen. Zum Beispiel wird alle 5 - 10 Minuten ein Durchschnitt der Daten verwendet, um ARG-Messwerte auf dem Smartphone zu erzeugen. Die Messwerte werden an den Server übertragen, und vom Server kommen die AH-Anzeigen auf das Smartphone. Das Ergebnis ist ein Diagramm der sich im Laufe der Zeit verändernden AH-Aktivität. Das ist für den Nutzer sehr wichtig. Dieses dynamische Bild der Veränderungen kann die Grundlage für die Vorhersage von Krisensituationen für einzelne Organe und Systeme des Körpers sein (Patent RU Nr. 2198600).

Die folgenden konkreten praktischen Beispiele für die Bewertung der Leistung von Wechselstromanlagen erläutert die vorgeschlagene Erfindung:

### Beispiel 1.

Versuchsperson RM, 32 Jahre alt, praktisch bei guter Gesundheit. 15 PGA-Messungen wurden zu verschiedenen Tageszeiten durchgeführt, Messungen mit Berechnung der Standardparameter der Phase und der spektralen Parameter der Pulswellenschätzung. Die Messungen wurden mit einem DPA v-2.0-Gerät (Meridian CO, LTD, Korea) im Vergleich zum Akabane-Test durchgeführt.

Beispiele sind hier seine Statgraf 6/0-Regressionsmodelle für die beiden Zweige (rechts und links) des ersten Lungenkanals (LUr, LU1) unter Verwendung der schrittweisen linearen Regression (Schrittweise Regression, Methode: Rückwärtsselektion, _F-zum-Einstieg: 4,0,_F-zum-Ausstieg: 4,0).

### Tabelle 2.

Korrelation von Pulswellenindikatoren mit dem rechten Ast des Pulmonalkanals. Multiple Regression - Abhängige Variable: Lur

**Tabelle 2**

| ***Parameter*** | ***Schätzung*** | ***Fehler*** | ***t*** | ***P-Wert*** |
|---|---|---|---|---|
| CONSTANT | -136,876 | 18,0929 | -7,56515 | 0,0001 |
| DDI | -16,4451 | 2,94081 | -5,59202 | 0,0005 |
| DEI | 0,407878 | 0,170826 | 2,38768 | 0,0440 |
| e_a | 28,5148 | 4,90534 | 5,81302 | 0,0004 |
| EI | 4,90983 | 0,720476 | 6,81471 | 0,0001 |
| Etc | 0,0663494 | 0,0109238 | 6,07383 | 0,0003 |
| HR | 0,501279 | 0,0897322 | 5,58638 | 0,0005 |
| LF | 0,0014523 | 0,000330727 | 4,39123 | 0,0023 |
| MeanNN | 0,0956981 | 0,0122489 | 7,81277 | 0,0001 |
| RMS_SD | -0,101564 | 0,0312083 | -3,25439 | 0,0116 |

| | | | | |
|---|---|---|---|---|
| R-Quadrat = 95,2316 Prozent R-Quadrat (bereinigt um d.f.) = 89,8672 Prozent Standardfehler der Schätzung = 0,47032 Mittlerer absoluter Fehler - 0,241865 | | | | |

Dieses 4-Stufen-Modell erklärt 95,23 % der Varianz mit einem mittleren absoluten Fehler (MAE) = 0,24. Dies ist für die Berechnungen durchaus akzeptabel. Die Polygonverteilung der beobachteten und vorhergesagten Ergebnisse ist ebenfalls gut verteilt.

### Tabelle 3.

Korrelation der Pulswellenindikatoren mit dem linken Ast des Lungenkanals von Patient 1.
Multiple Regression - Abhängige Variable: LUI

**Tabelle 3**

| *Parameter* | *Schätzung* | *Fehler* | *t* | *P-Wert* |
|---|---|---|---|---|
| CONSTAN T | 2,72207 | 0,674459 | 4,03594 | 0,0020 |
| DDI | -6,72737 | 2,11669 | -3,17825 | 0,0088 |
| DEI | 0,772629 | 0,165622 | 4,665 | 0,0007 |
| EI | 3,25521 | 0,704341 | 4,62163 | 0,0007 |
| PH | 0,126891 | 0,0405035 | 3,13284 | 0,0095 |
| SDNN | -0,0705396 | 0,0153518 | -4,59486 | 0,0008 |
| TP | 0,000904235 | 0,000180915 | 4,99812 | 0,0004 |

| | | | | |
|---|---|---|---|---|
| R-Quadrat = 92,09 Prozent R-Quadrat (bereinigt um d.f.) = 87,79 Prozent Standardfehler der Schätzung = 0,43 Mittlerer absoluter Fehler = 0,26 | | | | |

Dieses 12-Schritte-Modell erklärt 92,09 % der Varianz mit einem mittleren absoluten Fehler der Residuen (MAE)=0,26. Dies ist auch für die Berechnungen durchaus akzeptabel.

Die vorgeschlagene Methode, die auf der Schätzung von Pulswellen unter Verwendung von Standardparametern beruht, führte schließlich zu einer akzeptablen Schätzgenauigkeit für die 24 Hauptkanäle (Tabelle 4). Dabei schwankt die Schätzgenauigkeit mit einem R-Quadrat zwischen 78,74 und 99,35 % mit einem Standardfehlerwert (SE) von 0,29 bis 0,13 des als Kontrolle verwendeten Aqabane-Tests. Diese Modelle können also für weitere Berechnungen für diese Person verwendet werden. Mit zunehmender Anzahl der "Trainingsmaßnahmen" nimmt die Genauigkeit der Schätzung deutlich zu.

Insgesamt wurden signifikante Ergebnisse mit einem hohen Grad an Varianzaufklärung (R-Quadrat größer als 80 %) erzielt, einschließlich der geringen Zahl der durchgeführten Vergleiche.

**Tabelle 4.**

| ***Kanal*** | ***R-Quadrat %*** | ***SE*** | ***MSE*** |
|---|---|---|---|
| LUu | 95,2316 | 0,47032 | 0,241865 |
| LUI | 92,0995 | 0,439913 | 0,267999 |
| LIr | 85,5497 | 0,446997 | 0,256419 |
| LIl | 93,6198 | 0,618719 | 0,224814 |
| PCr | 98,517 | 0,181673 | 0,098615 |
| PCI | 95,182 | 0,296451 | 0,130852 |
| TEr | 96,9835 | 0,286557 | 0,175682 |
| TEl | 98,236 | 0,16301 | 0,081891 |
| HTr | 88,2439 | 0,358394 | 0,241076 |
| HTI | 78,7432 | 0,463017 | 0,299105 |
| SIr | 93,9816 | 0,400612 | 0,236069 |
| SIl | 84,0741 | 0,657689 | 0,344325 |
| SPr | 85,1375 | 0,336333 | 0,18218 |
| SPI | 95,6525 | 0,644805 | 0,319116 |
| LRr | 92,3039 | 0,468922 | 0,21705 |
| LRI | 99,356 | 0,550159 | 0,134014 |
| STr | 97,5002 | 0,493452 | 0,248938 |
| STI | 98,9596 | 0,160815 | 0,0782715 |
| GBr | 87,0933 | 0,690409 | 0,523139 |
| GBI | 97,208 | 0,629321 | 0,293873 |
| KIr | 82,887 | 1,15428 | 0,54468 |
| KIl | 81,356 | 1,91169 | 0,750065 |
| BLr | 95,5598 | 2,78782 | 1,54001 |
| BLI | 92,3946 | 3,17734 | 1,42006 |

Zusammenfassende Tabelle der schrittweisen linearen Regression der Beziehungen der Akupunkturkanäle mit den Phasen- und Spektralindizes der Pulswelle des Patienten 1

### Beispiel 2.

Patient BM, 69 Jahre alt, wurde zum Zeitpunkt der Rekrutierung für die statistische Auswertung durch schrittweise lineare Regression mit der Diagnose behandelt: Myokarditis, kompletter Linksschenkelblock, transienter A-V-Block 1-2st, einzelne ventrikuläre Extrasystolen.

Im Gegensatz zum vorherigen Fall mit Normalität waren mehr Beobachtungen (26) erforderlich, um Modelle mit einem hohen Varianzerklärungsgrad (R-Quadrat größer als 80 %) zu erhalten. Außerdem wurde die Zahl der verwendeten Parameter, die vom PDA-Instrument stammen, erhöht, einschließlich - neben der Norm - verschiedener anderer Phasenparameter des ARG, der Dauer der Segmente a,b,c,d,e und ihrer Verhältnisse. Darüber hinaus wurden beim Vergleich von EKG und ARG eine Reihe von Indikatoren (P-a, Q-a, R-a, S-a) verwendet, um bei Vorliegen einer Herzerkrankung wirksame Modelle zu erhalten.

Als Beispiel werden präsentiert (Tabelle 5, 6) Statgraf 6/0 Regressionsmodelle für die beiden Zweige (rechts und links) des ersten Lungenkanals (LUr, LU1) unter Verwendung schrittweiser linearer Regression (schrittweise Regression, Methode: Rückwärtsselektion, _F-to-enter: 4,0, F-to-remove: 4,0)

### Tabelle 5.

Korrelation der Pulswellenindikatoren mit dem rechten Ast des Lungenkanals von Patient 2.
Multiple Regression - Abhängige Variable: LUr

**Tabelle 5**

| ***Parameter*** | ***Schätzung*** | ***Fehler*** | ***t*** | ***P-Wert*** |
|---|---|---|---|---|
| CONSTANT | -448,376 | 122,994 | -3,64551 | 0,0108 |
| c_a | 12,1358 | 3,59955 | 3,37147 | 0,0150 |
| a_e | 0,0708608 | 0,0194354 | 3,64597 | 0,0108 |
| a_d | -0,167629 | 0,0406254 | -4,12621 | 0,0062 |
| a_b | 0,288699 | 0,0708219 | 4,07641 | 0,0065 |
| e_a | 45,6938 | 19,9809 | 2,28687 | 0,0622 |
| EEI | 6,58168 | 2,80275 | 2,34829 | 0,0572 |
| Etc | -0,0216481 | 0,0062752 | -3,44979 | 0,0136 |
| HF | 0,0444583 | 0,0113764 | 3,90795 | 0,0079 |
| HR | -1,80839 | 0,445241 | -4,06159 | 0,0066 |
| LF | -0,0275765 | 0,0078984 7 | -3,49137 | 0,0130 |
| LF_HF Ratio | 12,6595 | 3,74698 | 3,37858 | 0,0149 |
| P-a | 5,58528 | 1,41211 | 3,95526 | 0,0075 |
| R-a | 0,213927 | 0,058253 | 3,67238 | 0,0104 |
| TP | -0,00501954 | 0,0011621 1 | -4,31934 | 0,0050 |
| PH | -0,55026 | 0,154951 | -3,55119 | 0,0121 |

| | | | | |
|---|---|---|---|---|
| R-Quadrat = 86,36 Prozent R-Quadrat (bereinigt um d.f.) = 47,74 Prozent Standardfehler der Schätzung - 0,69 Mittlerer absoluter Fehler (MAE) = 0,28 | | | | |

Dieses 6-stufige Modell erklärt 86,36% der Varianz mit einem mittleren absoluten Fehler der Residuen (MAE)=0,28, was für Berechnungen durchaus akzeptabel ist.

### Tabelle 6.

Korrelation der Pulswellenindikatoren mit dem linken Ast des Lungenkanals von Patient 2
Multiple Regression - Abhängige Variable: Lui

**Tabelle 6**

| ***Parameter*** | ***Schätzung*** | ***Fehler*** | ***Statistik*** | ***P-Wert*** |
|---|---|---|---|---|
| CONSTANT | -3,59971 | 2,32993 | -1,54499 | 0,1463 |
| a_d | 0,0146243 | 0,00337046 | 4,33897 | 0,0008 |
| DEI | 24,2842 | 4,43025 | 5,48145 | 0,0001 |
| e_a | -13,7321 | 4,91749 | -2,79249 | 0,0152 |
| EI | -4,33713 | 1,29039 | -3,36111 | 0,0051 |
| HF | -0,0144778 | 0,00186888 | -7,74675 | 0,0002 |
| R-a | 0,171287 | 0,0392357 | 4,36558 | 0,0008 |
| LF | 0,00830727 | 0,00160147 | 5,18727 | 0,0002 |
| LF_HF Ratio | -5,19069 | 0,772727 | -6,71736 | 0,0009 |
| PH | 0,379408 | 0,0514498 | 7,37433 | 0,001 |
| TP | 0,00185493 | 0,000272393 | 6,80977 | 0,001 |

| | | | | |
|---|---|---|---|---|
| R-Quadrat = 90,05 Prozent R-Quadrat (bereinigt um d.f.) = 82,40 Prozent Standardfehler der Schätzung = 0,42 Mittlerer absoluter Fehler = 0,22 | | | | |

Dieses 12-Schritte-Modell erklärt 90,05 % der Varianz mit einem mittleren absoluten Fehler der Residuen (MAE)=0,22. Dies ist auch für die Berechnungen durchaus akzeptabel. Darüber hinaus wurde eine Reihe von Maßen verwendet, um effiziente Modelle für den Vergleich von EKG und ARG abzuleiten (R-a; P-a ).

Das Ergebnis ist auch eine akzeptable Genauigkeit für die Abschätzung der Akupunkturkanalaktivität. Dies spiegelt sich in der zusammenfassenden Tabelle 7 wider.

**Tabelle 7.**

| ***Kanal*** | ***R-Quadrat %*** | ***SE*** | ***MAE*** |
|---|---|---|---|
| LUr | 86,3692 | 0,696168 | 0,281971 |
| LUI | 90,0548 | 0,422496 | 0,226882 |
| LIr | 80,9447 | 1,07666 | 0,417587 |
| LIl | 82,1303 | 0,610152 | 0,23995 |
| PCr | 97,263 | 0,211793 | 0,0731053 |
| PCI | 98,996 | 0,0896887 | 0,0143439 |
| TEr | 94,8582 | 0,392752 | 0,145339 |
| TEl | 94,5687 | 0,370325 | 0,112726 |
| HTr | 99,3242 | 0,115044 | 0,0332176 |
| HTI | 88,4682 | 0,506223 | 0,179385 |
| SIr | 99,2364 | 0,0953893 | 0,026981 |
| SIl | 94,621 | 0,395383 | 0,12979 |
| SPr | 88,3817 | 0,707271 | 0,252576 |
| SPI | 93,0609 | 0,500097 | 0,184646 |
| LRr | 99,3809 | 0,099271 | 0,0349186 |
| LRI | 93,2393 | 0,642406 | 0,27141 |
| STr | 99,6304 | 0,124471 | 0,0388172 |
| STI | 85,564 | 0,486404 | 0,175184 |
| GBr | 96,2981 | 0,492625 | 0,194544 |
| GBI | 82,6845 | 0,231086 | 0,231086 |
| KIr | 98,9982 | 0,04117 | 0,014673 |
| KIl | 96,658 | 1,07765 | 0,372092 |
| BLr | 86,0694 | 0,59456 | 0,16363 |
| BLI | 97,9598 | 0,783703 | 0,297658 |

Zusammenfassende Tabelle der schrittweisen linearen Regression der Beziehungen der Akupunkturkanäle mit den Phasen- und Spektralindizes der Pulswelle von Patient 2.

Diese Beispiele zeigen, dass die Anwendung der vorgeschlagenen Methode, des Systems und des Moduls das beanspruchte technische Ergebnis erzielt. Die Methodik ist nämlich stark vereinfacht, da es keine kontinuierlichen aktiven Messungen der AC-Temperatur-Schmerzempfindlichkeitsschwellen nach dem Akabane-Test gibt. Um die AK-Aktivität nach der Reihe der "Lernmaßnahmen" zu beurteilen, genügt es, einen Finger auf den Pulssensor zu legen. Für Patienten mit kardiovaskulären Problemen ist es bei einigen Versionen des Systems auch möglich, ein EKG zu messen, während die beiden Elektroden am Gerätekörper gleichzeitig mit beiden Händen berührt werden. Auf diese Weise wird die Effizienz der Computerressourcen erheblich verbessert. Die Kosten für die Patientenausrüstung werden gesenkt, und die Funktionalität der Geräte wird verbessert. Zum Beispiel ist es möglich, diese Methode, das System und das Modul auch für die Schätzung, außer der Aktivität des AC, auch der grundlegenden Parameter der Hämodynamik durch die Berechnungsmethode zu verwenden.

### Liste der Referenzen:

1.Muzhikov, V.; Vershinina, E.; Belenky, V.; Muzhikov, R. Comparative Assessment of the Heart's Functioning by Using the Akabane Test and Classical Methods of Instrumental Examination Journal of Acupuncture and Meridian Studies, 10 (2017) pp. 171-179. https://doi.org/10.1016Zi.iams.2017.01.005).
2. Mujikov V.G. "Theorie und Praxis der Thermopunkturkanal-Diagnose und Behandlung". St. Petersburg, "Petrovsky Fund", 2000. S. 272.
3. Mujikov V. "Einführung in die Energoskopie des Menschen". Gopher Publishers nl. ISBN 90-5179-103-8, 2002, S.412.
4. Valery Muzhikov, Elena Vershinina, Ruslan Muzhikov. System zur Thermopunktur-Diagnostik und Überwachung von Patienten mit Typ-1-Diabetes. J Altern Complement Integr Med 2017, 3: pp.2-7 DOI: 10.24966/ ACIM-7562/100036
5. Valery Muzhikov, Elena Vershinina, Ruslan Muzhikov, Kirill Nikitin. Struktur der Interkanal- und Fünf-Primärelemente-Verbindungen nach dem Test von Akabane International Journal of Chinese Medicine Volume 2, Issue 3, September 2018, Pages: 18-29 , Received: Oct. 9, 2018; Akzeptiert: Okt. 30, 2018; Veröffentlicht: Dez. 3, 2018, DOI : 10.11648/i.ijcm.20180203.12 http://www.sciencepublishing roup.co m/ioumal/paperinfo?ioumalid=283&doi= 10.11648/j .ijcm.20180203.12)
6. Valery Muzhikov, Elena Vershinina, Vadim Belenky, Ruslan Muzhikov. Bewertung der Zusammenhänge zwischen anthropometrischen Daten und Akabane-Testergebnissen. Zeitschrift für Akupunktur und Meridianstudien. Februar 2018, Band 11, Ausgabe 1, Seiten 31 - 38. DOI: https://d0i.0rg/I 0.1016/j.jams.2018.01.0017.
7.Valery Muzhikov, Elena Vershinina und Ruslan Muzhikov. Möglichkeiten des Akabane-Tests zur Diagnose und Überwachung von Patienten mit Typ-2-Diabetes. Journal of Diabetes and Metabolism, 2018, 9:2 DOI: 10.4172/2155-6156.1000785
8. Portnov F.G. Elektropunktur-Reflextherapie. - Riga: Zinante, 1998.-P.103.
9. Muzhikov V.G. Theorie und Praxis der Thermopunkturkanal-Diagnostik und Behandlung. - SPb.: Petrovsky Fund Ltd, 2000,- S.27-37
10. http://csioumals.eom/IJCSC/PDF5-2/I.%20Sakshi.pdf
11.htt .7/tudr.tha ar.edu:8080/js ui/bitstream/I 0266/3182/4/3182.pdf
12. Mandeep Singh [1], Sakshi Bansal [2. Automatische Merkmalsextraktion in der Beschleunigungsplethysmographie. IJCSC, Band 5, Nr. 2, Sept. 2014, S. 1-9. ISSN-0973-7391 http://csioumals.eom/IJCSC/PDF5-2/I.%20Sakshi.pdf

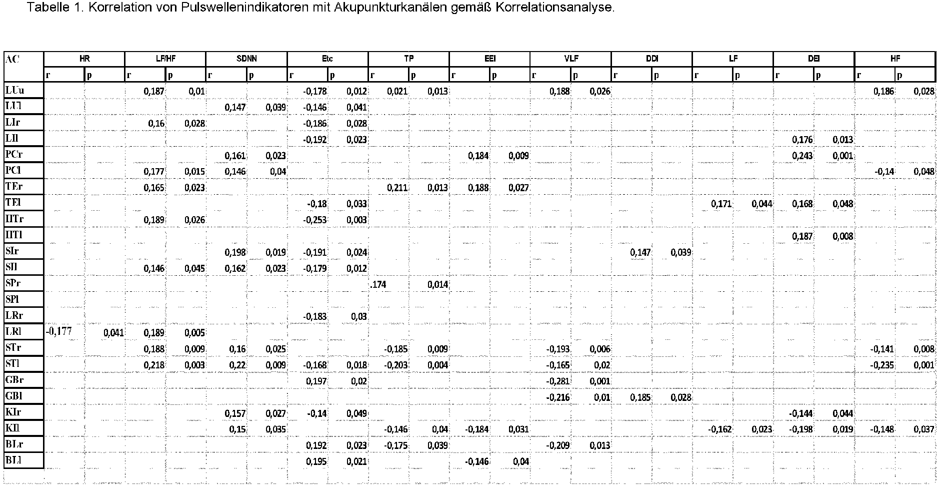

## Patentansprüche

1. Computerimplementiertes Verfahren der quantitativen Bewertung der Aktivität der Akkupunkturkanäle auf der Grundlage des Akabane Thermoakkupunkturtests, **dadurch kennzeichnet, dass** bei der Person, die durch die Methode der Fotoplethysmographie getestet wird, die Aufnahme der Pulswelle durchgeführt wird, dann werden die Schwellen deren Temperatur-Schmerzempfindlichkeit mit jeder Aufnahme im Laufe des Akabane Thermoakkupunkturtests gemessen, man formt aus der primären Pulswelle auf den diagnostischen Punkten der Akkupunkturkanäle synchron deren zweites beschleunigte Ableitung mit der Hervorhebung der standardmäßigen Wellen a, b, c, d, e, f mit der Berechnung deren zeitlichen und Amplitude-Charakteristiken und deren Beziehungen, wonach deren standardmäßigen Phasenwerte der Pulswelle SDNN, DEI, TP, Etc, EEI, DDI sowie spektrale Komponenten der Variabilität des Herzrythmus mit der Berechnung der Spektren LF, HF, VLF und deren Beziehungen, für jede konkrete Testperson wird aus einer Reihe der Paar-Stichproben der Werte der Pulswelle und des Thermoakkupunkturtests aufgrund der Methoden der mathematischen Analyse mit der Erstellung der Matrizen der Umrechnung aus den Pulswerten in Werte der Aktivität der Akkupunkturkanäle ein Modell deren Beziehungen erstellt, gefolgt von einer quantitativen Bewertung der Aktivität der Akkupunkturkanäle nach den Messergebnissen der Werte von nur der Pulswelle.

2. Das Verfahren nach dem Anspruch 1, das sich **dadurch kennzeichnet, dass** für Testpersonen, die Probleme mit Herz-Kreislauf haben, zusätzlich EKG-Werte abgenommen werden, die Zeitspannen zwischen P, Q, R EKG Zacken und der Welle a auf dem beschleunigten Fotoplethysmogramm festgestellt werden, wessen Daten im Weiteren während der Berechnungen bei der Erstellung der Matrizen der Umrechnung aus den Puls- und EKG-Werten in Werte der Aktivität der Akkupunkturkanäle berücksichtigt werden.

3. Das Verfahren nach dem Anspruch 2, das sich **dadurch kennzeichnet, dass** spektrale Werte der Pulswelle nach R Zacken auf EKG berechnet werden.

4. Das Verfahren nach dem Anspruch 2, das sich **dadurch kennzeichnet, dass** die durch die Cluster- und Faktoranalyse der Bearbeitung der großen Datenbanken gesammelten Statistiken von verschiedenen Testpersonen typische nach Struktur und Transformationskoeffizienten homogene Modelle der Verbindung der Puls- und EKG- Werte und Werte der Aktivität der Akkupunkturkanäle zur Reduzierung der Anzahl der individuellen Stichproben im Laufe der Testmessungen entdeckt werden, um die Effizienz der Methode zu verbessern.

5. Das Verfahren nach den Ansprüchen 1 und 2, das sich **dadurch kennzeichnet, dass** als Methode der mathematischen Analyse die Regressionsmethode oder die Methode der selbstlernenden neuronalen Netze verwendet wird.

6. Das Verfahren nach den Ansprüchen 1 und 2, das sich **dadurch kennzeichnet, dass** zur dynamischen Transformation mit Bedingungen der Lebensaktivität des Organismus zusätzliche Paar-Messungen für die Korrektion der Umrechnungsmatrizen mit der Berücksichtigung der neuen Angaben regelmäßig 1 b. 3 Mal pro Woche durchgeführt werden.

7. Das Verfahren nach den Ansprüchen 1 und 2, das sich **dadurch kennzeichnet, dass** die Aufnahme der Pulswelle von Ballen der Endphalangen der Finger oder Weichgewebe, zum Beispiel, im Bereich des Handgelenkes durchgeführt wird.

8. System der quantitativen Bewertung der Aktivität der Akkupunkturkanäle aufgrund des Akabane Thermopunkturtests konfiguriert, um das Verfahren nach Anspruch 1 auszuführen, welches das Modul zur Datenerfassung und - Verarbeitung beinhaltet, das so ausgeführt ist, dass die Schwellen der Temperatur-Schmerzempfindlichkeit der Akkupunkturpunkte des Eingangs-Ausgangs der Akkupunkturkanäle gemessen werden, den Server und das Steuerungs- und Kommutationsmodul beinhaltet, das durch bilaterale Datenübertragungskanäle mit dem Modul zur Datenerfassung und -Verarbeitung und dem Server verbunden ist, kennzeichnet sich dadurch, dass das Modul zur Datenerfassung und - Verarbeitung so ausgeführt ist, dass damit die Werte der Pulswelle registriert werden, dabei ist der Server so ausgeführt, dass damit das Modell der Beziehungen der erhaltenen Werte der Pulswelle und der Werte der Schwellen der Temperatur-Schmerzempfindlichkeit der Akkupunkturkanäle nach dem Akabane Test mit der Erstellung der Matrizen der Umrechnung aus den Pulswerten in die Werte der Aktivität der Akkupunkturkanäle erstellt werden, wobei das Steuerungs- und Kommunikationsmodul so ausgeführt ist, das es die Funktion der Datenaufnahme-Wiedergabe vom Modul zur Datenerfassung und -Verarbeitung und dem Server sowie der Bearbeitung, der Anzeige der Informationen und der Steuerung des Moduls zur Datenerfassung und -Verarbeitung und der Bearbeitung der Daten am Server ausführt.

9. Das System nach Anspruch 8, das sich **dadurch kennzeichnet, dass** das Modul zur Datenerfassung und -Verarbeitung so ausgeführt ist, dass damit EKG-Parameter zusätzlich registriert werden, dabei ist der Server so ausgeführt, dass ein Modell der Beziehungen der erhaltenen Werte der Pulswelle und EKG und der Werte der Schwellen der Temperatur-Schmerzempfindlichkeit der Akkupunkturkanäle nach dem Akabane Test mit der Erstellung der Matrizen der Umrechnung aus den Puls- und EKG-Werten in die Werte der Aktivität der Akkupunkturkanäle erstellt werden.

10. Das System nach Anspruch 9, das sich **dadurch kennzeichnet, dass** das Modul zur Datenerfassung und -Verarbeitung 2 Elektroden für EKG-Abnahme von Fingern an beiden Händen und einen Block zur EKG-Registrierung beinhaltet.

11. Das System nach Anspruch 10, das sich **dadurch kennzeichnet, dass** es einen zusätzlichen Sensor der Pulswelle beinhaltet, der als ein autonomes Modul ausgeführt ist, der durch bilaterale Verbindung mit dem Steuerungs- und Kommunikationsmodul und darüber mit dem Server verbunden ist und ins Gehäuse der Uhr oder in das Armband eingebaut ist mit einem System der Übertragung des Signals der Grafik der ersten Pulswelle auf das Steuerungs- und Kommunikationsmodul.

12. Das System nach Anspruch 11, das sich **dadurch kennzeichnet, dass** als Steuerungs- und Kommunikationsmodul ein Mobiltelefon, oder ein Tablet, oder ein personaler Computer verwendet wird.

13. Das System nach Anspruch 12, das sich **dadurch kennzeichnet, dass** das Modul zur Datenerfassung und -Verarbeitung als eine autonome Vorrichtung ausgeführt ist, auf deren Oberfläche ein IR-Strahler zur Bewertung der Schwellen der Schmerzempfindlichkeit nach dem Akabane Test, ein Sensor der Pulswelle, ein Bildschirm, eine USB-Schnittstelle zur Ladung der Batterien, Kippschalter und Schalter eingebaut sind.

14. Das System nach Anspruch 13, das sich **dadurch kennzeichnet, dass** das Modul zur Datenerfassung und -Verarbeitung zusätzlich 2 Elektroden zur EKG-Abnahme beinhaltet.

## Claims

1. A computer-implemented method for quantitative assessment of acupuncture channel activity based on the Akabane thermoacupuncture test, **characterised in that** a pulse wave recording is carried out on a person being tested by the method of photoplethysmography, then the thresholds of their temperature pain sensitivity are measured with each recording during the Akabane thermoacupuncture test, from the primary pulse wave at the diagnostic points of the acupuncture channels a second accelerated derivative thereof is synchronously formed with the extraction of the standard waves a, b, c, d, e, f with the calculation of their time and amplitude characteristics and their relationships, after which their standard phase values of the pulse wave SDNN, DEI, TP, Etc, EEI, DDI as well as spectral components of heart rate variability with the calculation of the spectra LF, HF, VLF and their relationships are calculated, for each specific test person a model of their relationships is created from a series of paired samples of the values of the pulse wave and the thermoacupuncture test based on methods of mathematical analysis with the creation of matrices for conversion from pulse values into values of acupuncture channel activity, followed by a quantitative assessment of acupuncture channel activity according to the measurement results of the values of only the pulse wave.

2. The method according to claim 1, **characterised in that** for test persons who have cardiovascular problems, ECG values are additionally recorded, the time intervals between P, Q, R ECG peaks and the wave a on the accelerated photoplethys-mogram are determined, the data of which are subsequently taken into account during the calculations when creating the matrices for conversion from pulse and ECG values into values of acupuncture channel activity.

3. The method according to claim 2, **characterised in that** spectral values of the pulse wave are calculated according to R peaks on the ECG.

4. The method according to claim 2, **characterised in that**
by cluster and factor analysis of the processing of large databases, collected statistics from various test persons, typical models homogeneous in structure and transformation coefficients of the connection of pulse and ECG values and values of acupuncture channel activity are identified for reducing the number of individual samples during test measurements in order to improve the efficiency of the method.

5. The method according to claims 1 and 2, **characterised in that** the regression method or the method of self-learning neural networks is used as the method of mathematical analysis.

6. The method according to claims 1 and 2, **characterised in that** for dynamic transformation with conditions of the vital activity of the organism, additional paired measurements for the correction of the conversion matrices taking into account the new data are carried out regularly 1 to 3 times per week.

7. The method according to claims 1 and 2, **characterised in that** the recording of the pulse wave is carried out from the sole of the distal phalanges of the fingers or soft tissue, for example, in the area of the wrist.

8. A system for quantitative assessment of acupuncture channel activity based on the Akabane thermopuncture test configured to carry out the method according to claim 1, which comprises a module for data acquisition and processing which is designed such that the thresholds of temperature pain sensitivity of the acupuncture points of the input-output of the acupuncture channels are measured, comprises a server and a control and communication module which is connected via bilateral data transmission channels to the module for data acquisition and processing and the server, **characterised in that** the module for data acquisition and processing is designed such that the values of the pulse wave are registered therewith, wherein the server is designed such that the model of the relationships of the obtained values of the pulse wave and the values of the
thresholds of temperature pain sensitivity of the acupuncture channels according to the Akabane test with the creation of the matrices for conversion from pulse values into the values of acupuncture channel activity are created therewith, wherein the control and communication module is designed such that it performs the function of data reception-reproduction from the module for data acquisition and processing and the server as well as the processing, the display of information and the control of the module for data acquisition and processing and the processing of data on the server.

9. The system according to claim 8, **characterised in that** the module for data acquisition and processing is designed such that ECG parameters are additionally registered therewith, wherein the server is designed such that a
model of the relationships of the obtained values of the pulse wave and ECG and the values
of the thresholds of temperature pain sensitivity of the acupuncture channels according to
the Akabane test with the creation of the matrices for conversion from pulse
and ECG values into the values of acupuncture channel activity are created.

10. The system according to claim 9, **characterised in that** the module for data acquisition and processing comprises 2 electrodes for ECG recording from fingers on both hands and a block for ECG registration.

11. The system according to claim 10, **characterised in that** it comprises an additional pulse wave sensor which is designed as an autonomous module which is connected via bilateral connection to the control and communication module and via this to the server and is integrated into the housing of a watch or into a wristband with a system for transmitting the signal of the graph of the primary pulse wave to the control and communication module.

12. The system according to claim 11, **characterised in that** a mobile telephone, or a tablet, or a personal computer is used as the control and communication module.

13. The system according to claim 12, **characterised in that** the module for data acquisition and processing is designed as an autonomous device, on the surface of which an IR emitter for assessing the thresholds of pain sensitivity according to the Akabane test, a pulse wave sensor, a screen, a USB port for charging the batteries, toggle switches and switches are installed.

14. The system according to claim 13, **characterised in that** the module for data acquisition and processing additionally comprises 2 electrodes for ECG recording.

## Revendications

1. Procédé mis en œuvre par ordinateur pour l'évaluation quantitative de l'activité des canaux d'acupuncture basé sur le test de thermo-acupuncture d'Akabane, **caractérisé en ce qu'**un enregistrement d'onde de pouls est effectué sur une personne testée par la méthode de photopléthysmographie, puis les seuils de sa sensibilité à la douleur thermique sont mesurés avec chaque enregistrement pendant le test de thermo-acupuncture d'Akabane, à partir de l'onde de pouls primaire aux points de diagnostic des canaux d'acupuncture une seconde dérivée accélérée de celle-ci est formée de manière synchrone avec l'extraction des ondes standard a, b, c, d, e, f avec le calcul de leurs caractéristiques temporelles et d'amplitude et de leurs relations, après quoi leurs valeurs de phase standard de l'onde de pouls SDNN, DEl, TP, Etc, EEI, DDI ainsi que les composantes spectrales de la variabilité de la fréquence cardiaque avec le calcul des spectres LF, HF, VLF et leurs relations sont calculées, pour chaque personne testée spécifique un modèle de leurs relations est créé à partir d'une série d'échantillons appariés des valeurs de l'onde de pouls et du test de thermo-acupuncture basé sur des méthodes d'analyse mathématique avec la création de matrices pour la conversion des valeurs de pouls en valeurs d'activité des canaux d'acupuncture, suivie d'une évaluation quantitative de l'activité des canaux d'acupuncture selon les résultats de mesure des valeurs de l'onde de pouls uniquement.

2. Procédé selon la revendication 1, **caractérisé en ce que** pour les personnes testées qui ont des problèmes cardiovasculaires, des valeurs d'ECG sont enregistrées en supplément, les intervalles de temps entre les pics P, Q, R de l'ECG et l'onde a sur le photopléthysmogramme accéléré sont déterminés, dont les données sont ensuite prises en compte lors des calculs lors de la création des matrices pour la conversion des valeurs de pouls et d'ECG en valeurs d'activité des canaux d'-acupuncture.

3. Procédé selon la revendication 2, **caractérisé en ce que** les valeurs spectrales de l'onde de pouls sont calculées selon les pics R sur l'ECG.

4. Procédé selon la revendication 2, **caractérisé en ce que** par analyse par grappes et analyse factorielle du traitement de grandes bases de données, des statistiques collectées auprès de diverses personnes testées, des modèles typiques homogènes en structure et des coefficients de transformation de la connexion des valeurs de pouls et d'ECG et des valeurs d'activité des canaux d'-acupuncture sont identifiés pour réduire le nombre d'échantillons individuels lors des mesures de test afin d'améliorer l'efficacité du procédé.

5. Procédé selon les revendications 1 et 2, **caractérisé en ce que** la méthode de régression ou la méthode des réseaux neuronaux auto-apprenants est utilisée comme méthode d'analyse mathématique.

6. Procédé selon les revendications 1 et 2, **caractérisé en ce que** pour une transformation dynamique avec les conditions de l'activité vitale de l'organisme, des mesures appariées supplémentaires pour la correction des matrices de conversion tenant compte des nouvelles données sont effectuées régulièrement 1 à 3 fois par semaine.

7. Procédé selon les revendications 1 et 2, **caractérisé en ce que** l'enregistrement de l'onde de pouls est effectué à partir de la plante des phalanges distales des doigts ou des tissus mous, par exemple, dans la zone du poignet.

8. Système pour l'évaluation quantitative de l'activité des canaux d'acupuncture basé sur le test de thermopuncture d'Akabane configuré pour mettre en œuvre le procédé selon la revendication 1, qui comprend un module d'acquisition et de traitement de données qui est conçu de telle sorte que les seuils de sensibilité à la douleur thermique des points d'acupuncture d'entrée-sortie des canaux d'acupuncture sont mesurés, comprend un serveur et un module de contrôle et de communication qui est connecté via des canaux de transmission de données bilatéraux au module d'acquisition et de traitement de données et au serveur, **caractérisé en ce que** le module d'acquisition et de traitement de données est conçu de telle sorte que les valeurs de l'onde de pouls sont enregistrées avec celui-ci, dans lequel le serveur est conçu de telle sorte que le modèle des relations des valeurs obtenues de l'onde de pouls et des valeurs des
seuils de sensibilité à la douleur thermique des canaux d'acupuncture selon le test d'Akabane avec la création des matrices pour la conversion des valeurs de pouls en valeurs d'activité des canaux d'acupuncture sont créés avec celui-ci, dans lequel le module de contrôle et de communication est conçu de telle sorte qu'il remplit la fonction de réception-reproduction de données du module d'acquisition et de traitement de données et du serveur ainsi que le traitement, l'affichage d'informations et le contrôle du module d'acquisition et de traitement de données et le traitement de données sur le serveur.

9. Système selon la revendication 8, **caractérisé en ce que** le module d'acquisition et de traitement de données est conçu de telle sorte que des paramètres d'-ECG sont enregistrés en supplément avec celui-ci, dans lequel le serveur est conçu de telle sorte qu'un
modèle des relations des valeurs obtenues de l'onde de pouls et de l'ECG et des valeurs
des seuils de sensibilité à la douleur thermique des canaux d'acupuncture selon le test d'Akabane avec la création des matrices pour la conversion des valeurs de pouls
et d'ECG en valeurs d'activité des canaux d'acupuncture sont créés.

10. Système selon la revendication 9, **caractérisé en ce que** le module d'acquisition et de traitement de données comprend 2 électrodes pour l'enregistrement d'ECG à partir des doigts des deux mains et un bloc pour l'enregistrement d'ECG.

11. Système selon la revendication 10, **caractérisé en ce qu'**il comprend un capteur d'onde de pouls supplémentaire qui est conçu comme un module autonome qui est connecté via une connexion bilatérale au module de contrôle et de communication et via celui-ci au serveur et est intégré dans le boîtier d'une montre ou dans un bracelet avec un système pour transmettre le signal du graphique de l'onde de pouls primaire au module de contrôle et de communication.

12. Système selon la revendication 11, **caractérisé en ce qu'**un téléphone mobile, ou une tablette, ou un ordinateur personnel est utilisé comme module de contrôle et de communication.

13. Système selon la revendication 12, **caractérisé en ce que** le module d'acquisition et de traitement de données est conçu comme un dispositif autonome, sur la surface duquel un émetteur IR pour évaluer les seuils de sensibilité à la douleur selon le test d'Akabane, un capteur d'onde de pouls, un écran, un port USB pour charger les batteries, des interrupteurs à bascule et des commutateurs sont installés.

14. Système selon la revendication 13, **caractérisé en ce que** le module d'acquisition et de traitement de données comprend en supplément 2 électrodes pour l'enregistrement d'ECG.
